Europäisches Patentamt

European Patent Office

Office européen des brevets

(11) Publication number: **0 211 424 B1**

(12) **EUROPEAN PATENT SPECIFICATION**

(45) Date of publication of patent specification: **08.05.91**

(51) Int. Cl.⁵: **C07F 1/08**, C07F 1/10, C07F 11/00, C07F 15/00, C07F 15/04, C07F 15/06, C07D 471/04, A61K 49/02

(21) Application number: 86110720.9

(22) Date of filing: 02.08.86

The file contains technical information submitted after the application was filed and not included in this specification

(54) **Metal-Isonitrile adducts for preparing radionuclide complexes.**

(30) Priority: **05.08.85 US 762392**

(43) Date of publication of application:
**25.02.87 Bulletin 87/09**

(45) Publication of the grant of the patent:
**08.05.91 Bulletin 91/19**

(84) Designated Contracting States:
**AT BE CH DE FR GB IT LI LU NL SE**

(56) References cited:
**EP-A- 163 294**
**EP-A- 0 150 844**
**EP-A- 0 183 555**

(73) Proprietor: **E.I. DU PONT DE NEMOURS AND COMPANY**
**1007 Market Street**
**Wilmington Delaware 19898(US)**

(72) Inventor: **Carpenter, Alan Purdom, Jr.**
**6 Davis Road**
**Tyngsboro Massachusetts 01879(US)**
Inventor: **Linder, Karen Elise**
**84 Hudson Street**
**Somerville Massachusetts 02143(US)**
Inventor: **Maheu, Leo John**
**11 Summer Street**
**Andover Massachusetts 01810(US)**
Inventor: **Patz, Michael Alan**
**4202 East Cactus Road Apartment No. 8301**
**Phoenix Arizona 85032(US)**
Inventor: **Thompson, Jeffery Scott**
**2211 Inwood Road**
**Wilmington Delaware 19810(US)**
Inventor: **Tulip, Thomas Hunt**
**13 Monza Road**
**Nashua New Hampshire 03060(US)**
Inventor: **Subramanyam, Vinayakam**
**1143 Gypsy Lane**
**Towson Maryland 21204(US)**

(74) Representative: **von Kreisler, Alek, Dipl.-Chem. et al**
**Deichmannhaus am Hauptbahnhof**
**W-5000 Köln 1(DE)**

## Description

This invention relates to a method for preparing isonitrile complexes of radionuclides, for example, of radioactive isotopes such as, $^{99m}Tc$, $^{99}Tc$. $^{97}Ru$, $^{51}Cr$, $^{57}Co$, $^{188}Re$, and $^{191}Os$, and particularly to metal-isonitrile adducts for making such radionuclide complexes.

## BACKGROUND OF THE INVENTION

A variety of radioisotope imaging and labeling agents have been developed in the past; however, certain of the materials previously available have generally suffered from the shortcomings of high cost, complexity of the method of preparation, or failure to exhibit high quality imaging or highly effective labeling.

Isonitrile complexes of various non-radioactive metals have been described. Oxime complexes of $^{99m}Tc$ have been described for use in labeling platelets. Wistow et al., J. Nucl. Med., Vol. 19, 483-487 (1978). The direct labeling of red blood cells with $^{99m}Tc$ by a reductive process, and the use of the labeled cells for imaging have been described. Smith et al., J. Nucl. Med., Vol. 17, 127-132 (1976). Various complexes of $^{99m}Tc$ with arsenic- and phosphorus-containing organic compounds have been proposed for use as imaging and labeling agents. Deutsch et al., Science, Vol. 214, 85-86 (1981); J. Nucl. Med., Vol. 22, 897-907 (1981): European Pat. Appln. No. 81400618.5, published Oct. 28, 1981, Publn. No. 0038756.

EP-A 0,163,294 relates to compositions for making technetium-labelled radiodiagnostic agents and, in particular, to soluble copper salt containing compositions for reducing technetium or accelerating the reduction of technetium for labelling such radiodiagnostic agents. The copper salt and ligand individually in admixture are lyophilized solids. The pertechnetate solution is added to a solution of the ligand and copper salt separately.

US-4,419,339 shows the preparation of Tc-ligand complexes by ligand exchange with a non-radioactive metal contained in a ligand complex of such a metal.

U.S. Patent No. 4,452,774 describes a coordination complex of an isonitrile ligand with a radioactive metal (radionuclide) selected from the class consisting of radioactive isotopes of Tc, Ru, Co, Pt, Fe, Os, Ir, W, Re, Cr, Mo, Mn, Ni, Rh, Pd, Nb, and Ta, and methods for using such complexes. Preferably, the isonitrile complexes comprise one of the above radioactive metals wherein each available coordination site is filled with an isonitrile ligand. The isonitrile ligand can be either monodentate or polydentate such as, for example, bidentate or tridentate. Also described is a kit comprising an isonitrile ligand and a reducing agent capable of reducing the radioactive metal to form the coordination complex.

Because of the general availability of supplies of $^{99m}Tc$ in clinical laboratories in the form of pertechnetate as well as the desirable half-life and gamma ray energy of this radionuclide, the complexes preferably contain $^{99m}Tc$, although complexes with other radionuclides are also described. Moreover, the general availability of supplies of pertechnetate make it convenient to use kits for preparation of various complexes of $^{99m}Tc$.

The isonitrile complexes can readily be prepared and isolated at both macro and tracer concentration in aqueous media, together with any of a wide variety of counterions, as appropriate. They display effective labeling characteristics for liposomes or vesicles, and a variety of living cells containing lipid membranes, and are also effective imaging agents for detecting abnormalities in the tissues of various organs, particularly in the heart, as well as the existence of blood clots. The complexes of $^{99m}Tc$ are particularly preferred because of the desirable nuclear properties of this radioisotopes, i.e., its half-life and gamma ray energy.

One problem encountered in preparing the isonitrile complexes described in U.S. 4,452,774 is that many isonitrile ligands are extremely volatile. Thus, the isonitrile ligand is difficult to handle, and lyophilized kits are not practical. Therefore, new and better ways for handling the isonitrile ligands for making radionuclide complexes are being sought.

## SUMMARY OF THE INVENTION

Certain of the above problems can be overcome by preparing soluble metal-adducts of isonitrile ligands and using the metal-isonitrile adduct to prepare the desired radionuclide-isonitrile complex. Surprisingly, metals that do form adducts of the isonitriles such as manganese, iron and ruthenium have not been successfully replaced from their isonitrile adducts (or salts) by, for instance, technetium to form the desired radionuclide complex with the isonitrile.

Therefore, the present invention provides a method for preparing a coordination complex of an isonitrile ligand and a radioactive metal selected from the class consisting of radioactive isotopes of Tc, Ru, Co, Pt, Fe, Os, Ir, W, Re, Cr, Mo, Mn, Ni, Rh, Pd, Nb and Ta, said method comprising admixing lyophilizate of a solid, water-soluble copper adduct of said isonitrile ligand with a radioactive metal in water, whereby said copper is replace with the radioactive metal thereby forming said coordination complex.


## DETAILED DESCRIPTION OF THE INVENTION

The Cu-metal-isonitrile adducts of the instant invention are reacted with a radioactive isotope selected from the list above to form the desired radionuclide complex.

Any isonitrile ligand can be used in the practice of this invention. Suitable isonitrile ligands include those having, for example, the formula CNR where the organic radical R can be aliphatic or aromatic and may be substituted with a variety of groups which may or may not be charged. Among the aromatic R groups which may be present are phenyl, tolyl, xylyl, naphthyl, biphenyl and substituted aromatic groups containing such substitutents as halo (e.g., chloro, bromo, iodo or fluoro), hydroxy, nitro, alkyl, alkyl ether, alkyl ester, etc.; among the aliphatic R groups which may be present are alkyl, preferably containing 1 to 20 carbon atoms, such as methyl, ethyl, n-propyl, isopropyl, n-butyl, t-butyl, isobutyl, n-hexyl, 2-ethylhexyl, dodecyl, stearyl, etc. Substituent groups may also be present in the aliphatic groups, including among others the same substituent groups as those listed above for aromatic groups.

The lipophilic characteristics of the complex can be varied by varying the R groups to adapt the complex for labeling selected materials, for membrane transport such as for the blood-brain barrier, or for imaging selected organs and dynamic processes related to their function (e.g. heart function).

The Cu-metal-isonitrile adducts of the present invention can readily be prepared by admixing a complex of the displaceable Cu-metal and the isonitrile ligand in a suitable media at temperatures from room temperature to reflux temperature or even higher. The reaction is generally complete after about 1 minute to about 2 hours, depending upon the reagents employed and the conditions used.

Any desired counterion may also be present in the composition, such as nitrate, sulfate, chloride, fluoride, bromide, iodide, tetrafluoroborate, hexafluorophosphate, trifluoromethanesulfonate, etc.

For isonitriles whose Cu-metal adducts can only be isolated as oils, the preferred Cu-metal-isonitrile adducts are those where at least two of the isonitrile ligands are replaced by a single multidentate chelating ligand. These are preferred for ease of handling because of their greater tendency to form solid materials. Examples of bidentate chelating ligands include phenanthroline, substituted phenanthrolines, diimines, substituted diimines, bipyridine, substituted bipyridines, and related chelating ligands. Such mixed ligand adducts would have the general formula $[Cu(CNR)_2Y]X$, where R is defined as above, Y is the bidentate chelating ligand, and X is an appropriate counterion.

The desired labeled radionuclide complexes are prepared from the Cu-metal-isonitrile adducts by admixing the adduct with the radioactive metal in suitable media at temperatures from room temperature to reflux temperatures or even higher. The desired labeled isonitrile complexes are isolable and can be obtained in high yields. In some cases the Cu-metal-isonitrile adduct can itself act as a reducing agent thus eliminating the need for an additional reducing agent. Additional reducing agents, when required or desired to speed up the reaction, are well known to those skilled in the art. Examples of such well-known reducing agents include stannous salt (often used in the form of kits), formamidine sulfinic acid, sodium dithionite, sodium bisulfite, hydroxylamine, ascorbic acid, and the like. The reaction is generally complete after about 1 minute to about 2 hours, depending upon the particular reagents employed and the conditions used. The yield of labelled radionuclide isonitrile complex is about 45%, preferably at least 70%, and more preferably at least 90% of the radioactive nuclide used for labeling.

In the case of technetium such as, for example $^{99}Tc$ or $^{99m}Tc$, an isonitrile complex is preferably made by mixing an appropriate reducing agent (capable of reducing technetium in aqueous medium) and the Cu-metal-isonitrile adduct, then adding pertechnetate. Alternatively, the Cu-metal adduct and pertechnetate are mixed, then reductant added.

The isonitrile technetium complexes prepared in accord with this invention can also be prepared from preformed technetium complexes having oxidation states for technetium of, for instance, III, IV or V, by treating these preformed complexes with an excess of Cu-metal-isonitrile adduct under suitable conditions. For example, the technetium-isonitrile complex can also be prepared by reacting the desired metal-isonitrile adduct with the hexakis-thiourea complex of $Tc^{III}$ or with a technetium glucoheptonate complex, or the like.

An excess of the Cu-metal-isonitrile adduct, up to 1 to 100 fold molar excess or more, and an excess of reducing agent, can be used in the complexing reaction to ensure maximum yield from the technetium. Following the reaction, the desired complex can be separated from the reaction mixture, if required, for example, by crystallization or precipitation or by conventional chromatography or ion exchange chromatography (see U.S. 4,452,774).

The invention further relates to a kit for preparing a coordination complex of an isonitrile ligand and a radionuclide selected from the class consisting of a radioactive isotope of Tc, Ru, Co, Pt, Fe, Os, Ir, W, Re, Cr, Mo, Mn, Ni, Rh, Pd, Nb and Ta, said kit comprising a lyophilisate of a predetermined quantity of (a) an adduct of a complex of copper and said isonitrile ligand and (b) a predetermined quantity of a reducing agent capable of reducing a predetermined quantity of a preselected one of said radionuclides to form said complex by replacing the copper from said ligand with said radionuclide.

It is also preferred that the isonitrile ligand and reducing agent be lyophilized, when possible, to facilitate storage stability. If lyophilization is not practical, the kits are stored frozen. The Cu-metal-isonitrile adduct and reducing agent are preferably contained in sealed, non-pyrogenic, sterilized containers.

In one embodiment of the invention, a kit for use in making the radionuclide complexes in accord with the present invention from a supply of $^{99m}$Tc such as the pertechnetate solution in isotonic saline (available in most clinical laboratories) includes the desired quantity of a selected isonitrile ligand in the form of Cu-metal-isonitrile adduct to react with a predetermined quantity of pertechnetate, and a predetermined quantity of reducing agent such as, for example, stannous ion in the form of stannous glucoheptonate to reduce the predetermined quantity of pertechnetate to form the desired technetium-isonitrile complex.

Isonitrile complexes prepared in accord with this invention can be used to label human erythrocytes (red blood cells); to measure lung function; to measure hepatobiliary function; and for myocardial imaging. For instance, both t-butyl and isopropyl isonitrile products have been used to visualize myocardial tissue by external imaging.

Cells can be readily labeled by incubating the radiolabeled complexes of this invention with such cells in a suitable medium and measuring the uptake of radioactivity in accord with the methods described by Kassis, A.I. et al., J. Nucl. Med., Vol. 21, 88-90 (1980). Incorporation of the radioactive complex can be as high as 29 pCi/cell. Studies have shown that the radioactive label can be 90% retained for up to sixteen hours. Autologous leukocytes separated from fresh rabbit blood were labeled with the $^{99m}$Tc complex and subsequently reinjected into the rabbit. The distribution of the radiolabeled cells could be followed by gamma camera.

The choice of radionuclides will depend on the use. For example, preferred radionuclides for diagnostic imaging are radioactive isotopes of Tc, Ru, Co, Pt, Fe, Os, and Ir; preferred radionuclides for therapeutic uses are radioactive isotopes of W, Re, Fe, and Os; preferred radionuclides for radioactive tagging are Cr, Mo, Co, Tc, Fe, Mn, W, Ru, Ni, Rh, Ir, Pd, Nb, and Ta.

EXAMPLES

Example 1 - Preparation of [Cu(CN-t-Bu)$_4$]NO$_3$

Under an inert atmosphere, 2.64g (10.9 mmol) cupric nitrate, Cu(No$_3$)$_2$3H$_2$O, and 2.10g (33.0 mmol) copper dust are stirred in 25 mL predried methanol until dissolved; 10 mL (88.4 mmol) of t-butyl isonitrile is added and the mixture stirred for four hours. Excess copper metal is removed by filtration under nitrogen and then the solvent is removed under high vacuum. The resulting solid is dissolved in 10 mL methanol and filtered. The product is precipitated with the addition of diethyl ether. The white solid is dried in vacuo at ambient temperature to produce the desired complex in 65% yield.

Example 2 - Preparation of [Cu(CN-t-Bu)$_4$ ]Cl

Under dry N$_2$ or an appropriate inert atmosphere, a suspension of 1.0 g (10.1 mmol) of CuCl in 100 mL of CHCl$_3$ is treated with 6.8 mL (5.0g, 60.1 mmol) of t-butyl isonitrile. The mixture warms slightly and is allowed to stir for 1 hour. The solution is then filtered to remove any unreacted CuCl, and the CHCl$_3$ solution reduced to 10-15 mL on a rotary-evaporator. To the reduced solution is then added 75-100 mL of diethyl ether (anhydrous). The colorless solid is then isolated by filtration on a glass frit. The isolated solid is then washed with diethyl ether (anhydrous) 3 x 50 mL, and allowed to dry. The yield of the desired product is about 75%.

Example 10 - Preparation of [Cu(1-isocyano-2-methoxy-2 methylpropane)$_2$-(1,10-phenanthroline)-BF$_4$

To a 100 mL round bottom flask is added 0.251 g (0.798 mmol) of [Cu(CH$_3$CN)$_4$]BF$_4$ and 10 mL of tetrahydrofuran under a nitrogen atmosphere. To this suspension is added 221 μL (199 mg, 1.76 mmol) of 1-isocyano-2-methoxy-2-methylpropane with stirring. After stirring at 25° C for 30 min, all solids dissolve. A solution of 158 mg (0.798 mmol) of 1,10-phenanthroline in 2 mL, of tetrahydrofuran is then added dropwise during which time the color of the reaction mixture turns yellow and a fine microcrystalline precipitate appears. After stirring for 2 hr, 50 mL of diethyl ether is slowly added and the product is filtered, washd with diethyl ether (2 x 10 mL) and dried for 24 hr under vacuum. Yield: 0.418 g (94%).

Example 4 - Preparation of [Cu(2-isocyano-1-methoxy-propane)₂ bathophenanthroline disulfonic acid disodium salt]Cl•H₂O

To a 100 mL round bottom flask is added 155 mg (1.57 mmol) of CuCl and 5 mL of methanol under a nitrogen atmosphere. To this slurry is added 380 µL, (342 mg, 3.45 mmol) of 2-isocyano-1-methoxypropane dropwise. The mixture is stirred for 15 min and all solids dissolve. A solution of 840 mg (1.57 mmol) of bathophenanthroline disulfonic acid, disodium salt in 15 mL of methanol is then added dropwise and the mixture is allowed to stir for 3 hr during which time the color of the solution turns yellow. Diethyl ether (ca. 50 mL) is then slowly added to precipitate the product as a fine yellow powder which is filtered, washed with diethyl ether (2 x 10 mL), and dried for 24 hr under vacuum. Yield: 1.186 g (89%).

**Claims**

1. A method for preparing a coordination complex of an isonitrile ligand and a radioactive metal selected from the class consisting of radioactive isotopes of Tc, Ru, Co, Pt, Fe, Os, Ir, W, Re, Cr, Mo, Mn, Ni, Rh, Pd, Nb and Ta, said method comprising admixing a solid, water-soluble copper adduct of said isonitrile ligand with a radioactive metal in water, whereby said copper is replaced with the radioactive metal thereby forming said coordination complex.

2. The method of claim 1 wherein said radioactive metal is Tc.

3. The method of claim 2 wherein said radioactive metal is Tc-99m.

4. The method of claim 2 wherein the isonitrile ligand has the formula CNR where R is butyl.

5. The method of claim 2 wherein the isonitrile ligand has the formula CNR where R is butyl having an alkyl ether or alkyl ester substitution.

6. The method of Claim 1 or 2 wherein the copper adduct of said isonitrile ligand has the formula [Cu(CNR)₂Y]X wherein R is a suitable organic radical, X is an appropriate counter ion and Y is a bidentate chelating ligand.

7. The method of Claim 6 wherein Y is selected from the group consisting of phenanthroline, substituted phenanthroline, diimine, substituted diimine, bipyridine or substituted bipyridine.

8. A kit for preparing a coordination complex of an isonitrile ligand and a radionuclide selected from the class consisting of a radioactive isotope of Tc, Ru, Co, Pt, Fe, Os, Ir, W, Re, Cr, Mo, Mn, Ni, Rh, Pd, Nb and Ta, said kit comprising a lyophilisate of a predetermined quantity of (a) an adduct of a complex of copper and said isonitrile ligand and (b) a predetermined quantity of a reducing agent capable of reducing a predetermined quantity of a preselected one of said radionuclides to form said complex by replacing the copper from said ligand with said radionuclide.

9. A kit as claimed in claim 8 wherein said lyophilized isonitrile adduct and said reducing agent are each contained in a sealed, sterilized container.

10. The kit of claim 8 or 9 wherein the isonitrile ligand has the formula CNR where R is butyl.

11. The kit of claim 8 or 9 wherein the isonitrile ligand has the formula CNR where R is butyl having an alkyl ether or alkyl ester substitution.

12. A kit of Claim 8 or 9 wherein the copper adduct of said isonitrile ligand has the formula [Cu(CNR)₂Y]X wherein R is a suitable organic radical, X is an appropriate counter ion and Y is a bidentate chelating ligand.

13. A kit of Claim 12 wherein Y is selected from the group consisting of phenanthroline, substituted phenanthroline, diimine, substituted diimine, bipyridine or substituted bipyridine.

**Revendications**

1. Un procédé pour préparer un complexe de coordination d'un ligand isonitrile et d'un métal radioactif choisi dans la classe comprenant les isotopes radioactifs de Tc, Ru, Co, Pt, Fe, Os, Ir, W, Re, Cr, Mo, Mn, Ni, Rh, Pd, Nb et Ta, ce procédé comprenant le mélange d'un lyophilisat d'un produit d'addition solide, soluble dans l'eau, de cuivre et de ce ligand isonitrile avec un métal radioactif dans de l'eau, si bien que ce cuivre est remplacé par le métal radioactif et que cela forme ce complexe de coordination.

2. Le procédé suivant la revendication 1, dans lequel ce métal radioactif est Tc.

3. Le procédé suivant la revendication 2, dans lequel ce métal radioactif est $^{99m}$Tc.

4. Le procédé suivant la revendication 2, dans lequel le ligand isonitrile est représenté par la formule CNR dans laquelle R est un groupe butyle.

5. Le procédé suivant la revendication 2, dans lequel le ligand isonitrile est représenté par la formule CNR dans laquelle R est un groupe butyle substitué par un groupe éther alkylique ou ester alkylique.

6. Le procédé suivant la revendication 1 ou la revendication 2, dans lequel le produit d'addition de cuivre et de ce ligand isonitrile est représenté par la formule [Cu[CNR]₂Y]X, laquelle R est un radical organique approprié, X est un contre-ion approprié et Y est un ligand chélatant bidentate.

7. Le procédé suivant la revendication 6, dans lequel Y est choisi dans le groupe comprenant une phénanthroline, une phénanthroline substituée, une diimine, une diimine substituée, une bipyridine ou une bipyridine substituée.

8. Une trousse pour préparer un complexe de coordination d'un ligand isonitrile et d'un radionucléide choisi dans la classe comprenant un isotope radioactif de Tc, Ru, Co, Pt, Fe, Os, Ir, W, Re, Cr, Mo, Mn, Ni, Rh, Pd, Nb et Ta, cette trousse comprenant un lyophilisat d'une quantité prédéterminée : (a) d'un produit d'addition d'un complexe de cuivre et de ce ligand isonitrile et (b) d'une quantité prédéterminée d'un agent réducteur capable de réduire une quantité prédéterminée d'un radionucléide présélectionné parmi ces radionucléides pour former ce complexe par remplacement du cuivre de ce ligand par ce radionucléide.

9. La trousse suivant la revendication 8, dans laquelle ce produit d'addition d'isonitrile lyophilisé et cet agent réducteur sont contenus chacun dans un récipient stérilisé, fermé hermétiquement.

10. La trousse suivant la revendication 8 ou la revendication 9, dans laquelle le ligand isonitrile est représenté par la formule CNR dans laquelle R est un groupe butyle.

11. La trousse suivant la revendication 8 ou la revendication 9, dans laquelle le ligand isonitrile est représenté par la formule CNR dans laquelle R est un groupe butyle substitué par un groupe éther alkylique ou ester alkylique.

12. La trousse suivant la revendication 8 ou la revendication 9, dans laquelle le produit d'addition de cuivre et de ce ligand isonitrile est représenté par la formule [Cu(CNR)₂Y]X, dans laquelle R est un radical organique approprié, X est un contre-ion approprié et Y est un ligand chélatant bidentate.

13. La trousse suivant la revendication 12, dans laquelle Y est choisi dans le groupe comprenant une phénanthroline, une phénanthroline substituée, une diimine, une diimine substituée, une bipyridine ou une bipyridine substituée.

**Ansprüche**

1. Verfahren zur Herstellung eines Koordinations-Komplexes eines Isonitril-Liganden und eines radioaktiven Metalls, das aus der aus radioaktiven Isotopen von Tc, Ru, Co, Pt, Fe, Os, Ir, W, Re, Cr, Mo, Mn, Ni, Rh, Pd, Nb und Ta bestehenden Klasse ausgewählt ist, umfassend das Vermischen eines festen, wasserlöslichen Kupfer-Addukts des Isonitril-Liganden mit einem radioaktiven Metall in Wasser, wobei das Kupfer durch das radioaktive Metall ersetzt wird und dadurch der Koordinations-Komplex gebildet wird.

2. Verfahren nach Anspruch 1, worin das radioaktive Metall Tc ist.

3. Verfahren nach Anspruch 2, worin das radioaktive Metall Tc-99m ist.

4. Verfahren nach Anspruch 2, worin der Isonitril-Ligand die Formel CNR hat, in der R Butyl ist.

5. Verfahren nach Anspruch 2, worin der Isonitril-Ligand die Formel CNR hat, in der R Butyl mit einer Alkylether- oder Alkylester-Substitution ist.

6. Verfahren nach Anspruch 1 oder 2, worin das Kupfer-Addukt des Isonitril-Liganden die Formel [Cu(CNR)₂Y]X hat, worin R ein geeigneter organischer Rest ist, X ein geeignetes Gegenion ist und Y ein zweizähniger chelatbildender Ligand ist.

7. Verfahren nach Anspruch 6, worin Y aus der aus Phenanthrolin, substituiertem Phenanthrolin, Diimin, substituiertem Diimin, Bipyridin oder substituiertem Bipyridin bestehenden Gruppe ausgewählt ist.

8. Kit zur Herstellung eines Koordinations-Komplexes eines Isonitril-Liganden und eines Radionuklids, das aus der aus radioaktiven Isotopen von Tc, Ru, Co, Pt, Fe, Os, Ir, W, Re, Cr, Mo, Mn, Ni, Rh, Pd, Nb und Ta bestehenden Klasse ausgewählt ist, umfassend ein Lyophilisat einer vorher festgelegten Menge (a) eines Addukts eines Komplexes aus Kupfer und dem Isonitril-Liganden und (b) eine vorher festgelegte Menge eines Reduktionsmittels, die eine vorher festgelegte Menge eines vorher aus den genannten Radionukliden ausgewählten Radionuklids zu reduzieren vermag, um den Komplex dadurch zu bilden, daß das Kupfer durch das Radionuklid ersetzt wird.

9. Kit nach Anspruch 8, worin das lyophilisierte Isonitril-Addukt und das Reduktionsmittel jeweils in einem abgedichteten sterilisierten Behälter enthalten sind.

10. Kit nach Anspruch 8 oder 9, worin der Isonitril-Ligand die Formel CNR hat, in der R Butyl ist.

11. Kit nach Anspruch 8 oder 9, worin der Isonitril-Ligand die Formel CNR hat, in der R Butyl mit einer Alkylether- oder Alkylester-Substitution ist.

12. Kit nach Anspruch 8 oder 9, worin das Kupfer-Addukt des Isonitril-Liganden die Formel [Cu-(CNR)$_2$Y]X hat, worin R ein geeigneter organischer Rest ist, X ein geeignetes Gegen-Ion ist und Y ein zweizähniger chelatbildender Ligand ist.

13. Kit nach Anspruch 12, worin Y aus der aus Phenanthrolin, substituiertem Phenanthrolin, Diimin, substituiertem Diimin, Bipyridin oder substituiertem Bipyridin bestehenden Gruppe ausgewählt ist.